# EUROPÄISCHE PATENTSCHRIFT

(11) **EP 3 206 017 B1**
(45) Veröffentlichungstag und Bekanntmachung des Hinweises auf die Patenterteilung: **12.09.2018**
(21) Anmeldenummer: 16154849.0
(22) Anmeldetag: 09.02.2016
(51) Int. Cl.: G01N 21/63, F02D 41/00, G01N 1/22, G01N 21/67, G01N 21/71, G01N 21/76, F23N 5/00, G01N 33/22

(54) **SENSOR UND VERFAHREN ZUR BESTIMMUNG DER LUFTZAHL EINES BRENNGAS-LUFT-GEMISCHES**
SENSOR AND METHOD FOR DETERMINING THE AIR RATIO OF A COMBUSTIBLE GAS-AIR MIXTURE
CAPTEUR ET DISPOSITIF DE DETERMINATION DU COEFFICIENT D'AIR D'UN MELANGE GAZ COMBUSTIBLE/AIR

(43) Veröffentlichungstag der Anmeldung: 16.08.2017
(73) Patentinhaber: Elster GmbH, 55252 Mainz-Kastel (DE)
(72) Erfinder: Leerkotte, Bernardus Johannes Maria, 7572 Oldenzaal (NL); Schäfer, Christian Franz Hugo, 49080 Osnabrück (DE)
(74) Vertreter: Zenz Patentanwälte Partnerschaft mbB

(56) Entgegenhaltungen:
- EP-A1- 0 156 200
- EP-A2- 1 591 723
- WO-A1-2015/054323
- CN-A- 104 823 041
- DE-A1- 4 121 924
- FR-A1- 2 816 056
- US-A1- 2003 003 590

## Beschreibung

Die Erfindung betrifft einen Sensor zur Bestimmung der Luftzahl eines Brenngas-Luft-Gemisches und ein zugehöriges Verfahren.

Die Messung der Luftzahl ist in zahlreichen Verbrennungsverfahren äußerst wichtig, insbesondere um Verbrennungsprozesse effizient und schadstoffarm ablaufen zu lassen. Im Bereich der Fahrzeugtechnik sind hierzu die sogenannten Lambda(λ)-Sonden bekannt, welche meist auf dem Prinzip elektrochemischer Messzellen basieren. Ein Beispiel für einen solchen Sensor ist der WO 2009 144051 A1 zu entnehmen.

Derartige Systeme sind bei Veränderungen im Gemischverhältnis und bei Veränderungen in der Größe des Massenstroms limitiert hinsichtlich ihrer Reaktionszeit sowie ihrer massenstromabhängigen Totzeit und sind häufig auch recht teuer in der Fertigung.

Das Dokument EP 0 156 200 A1 offenbart ein Verfahren und eine Anordnung zur Gemischbestimmung eines Brennstoffgemisches. Ein Teil des Brennstoffgemisches wird vor einem Brennraum abgezweigt und in einer Nebenverbrennungseinrichtung zur Reaktion gebracht. Die Verbrennungsprodukte der Nebenverbrennung werden analysiert und daraus wird ein Signal für die Einstellung des Mischungsverhältnisses bestimmt.

Die CN 104 823 041 A beschreibt ein anderes System zur Gemischbestimmung. Das System hat einen Brenner mit einer zugeordneten Brennkammer. Ein Kraftstoffmesssensor misst die Kraftstoffmenge, die der Brennkammer zugeführt wird. Ein Betriebsmodul empfängt ein Umschaltsignal von einem Analysemodul, welches mit einem optischen Sensor gekoppelt ist, um das Innere der Brennkammer zu überwachen und abhängig davon die Luftmenge einzustellen.

Aus der WO 2015/054323 A1 ist ein Vormischbrenner bekannt. Der Brenner hat eine Vormischkammer, ein perforierter Reaktionshalter ist innerhalb des Kammervolumens positioniert. Am Halter erfolgt eine Verbrennungsreaktion, wenn ein Teil des Oxidationsmittel-Brennstoff-Gemisches verbrennt. Zwischen der Vormischkammer und dem Halter ist eine Flammensperre angeordnet, wobei die Vormischkammer eine Öffnung aufweist, durch die das Oxidationsmittel-Brennstoff-Gemisch strömt. Das System begrenzt den Oxidationsmitteleinlass auf einen Winkel, der ankommenden Brennstoff und Oxidationsmittel von der Öffnung wegleitet, wodurch das Mischen des Brennstoffs und des Oxidationsmittels verbessert wird.

Aufgabe der Erfindung ist es, eine Erfassung der Luftzahl bei günstigen Kosten, rascher Reaktionszeit und kurzer Totzeit bereitzustellen.

Diese Aufgabe wird gelöst durch einen Sensor mit den Merkmalen des Patentanspruches 1 sowie ein Verfahren mit den Merkmalen des Patentanspruches 9.

Der erfindungsgemäße Sensor und das erfindungsgemäße Verfahren bedienen sich eines optischen Messverfahrens zur Ermittlung der Luftzahl. Dazu wird ein begrenzter Messraum gebildet, in welchem ein Brenngas-Luft-Gemisch eindiffundieren kann. Erfindungsgemäß wird ein geringer Anteil eines vorbeiströmenden Brenngas-Luft-Gemisches abgezweigt, wovon wiederum ein kleiner Anteil an einer kontrollierten Reaktion teilnimmt. Diese Reaktion wird optisch überwacht und die optischen Signale werden ausgewertet.

Der Messraum des Sensors, in welchen das Brenngas-Luft Gemisch zur Reaktion und Messung eindiffundiert, ist durch ein Gehäuse begrenzt, wobei ein diffusionsoffener Zugang zu einem vorbeiströmenden Brenngas-Luft-Gemisch bereitgestellt ist. Beispielsweise kann ein erfindungsgemäßer Sensor in einem Gas-Brennwertgerät hinter dem Mischbereich angeordnet sein, wobei der diffusionsoffene Zugang an den Strömungsweg des Brenngas-Luft-Gemisches grenzt.

Die Diffusionspassage kann vielfältig gestaltet sein, wobei der rasche Stoffaustausch mit dem vorbeiströmenden Brenngas-Luft-Gemisch gewährleistet sein soll. Andererseits dient die Diffusionspassage als Flammensperre, da in dem Messraum chemische Reaktionen ablaufen und eine Flammentrennung von dem vorbeiströmenden Brenngas-Luft-Gemisch erforderlich ist, auch wenn im Messraum bei ordnungsgemäßem Betrieb keine Flammenbildung erfolgt. Entsprechend sind Trennmittel in der Diffusionspassage mit einer Eignung als Flammensperre ausgebildet und beispielsweise in Gestalt eines Gitters oder metallischen oder keramischen Netzes oder auch einer gesinterten Einlage oder Fritte geformt.

Durch Diffusionsprozesse findet jederzeit ein Stofftransport zwischen dem Messraum in dem Gehäuse und dem jenseits der Trennmittel vorbeiströmenden Brenngas-Luft-Gemisch statt. Die Diffusionsöffnungen im Trennmittel sind dabei so klein gewählt, dass Turbulenzen in dem vorbeiströmenden Brenngas-Luft-Gemisch nicht in die Messkammer übertragen werden.

Innerhalb des Messraums ist ein elektrisch betriebenes Anregungsmittel angeordnet. Unter elektrisch betriebenen Anregungsmitteln ist in diesem Zusammenhang jede Einrichtung zu verstehen, welche eine Energiezufuhr (oder auch Energieeintrag) in den Messraum bewirken kann, um eine chemische Reaktion eines Brenngas-Luft-Gemisches in dem Messraum herbeizuführen. Entsprechend können die elektrisch betriebenen Anregungsmittel Heizmittel oder Zündmittel sein. In jedem Fall wird von dem Anregungsmittel Energie gezielt in den Messraum eingebracht. Die Anregungsmittel sind in oder an dem Messraum so platziert und ausgerichtet, dass der Energieeintrag an einer im Messraum befindlichen Reaktionsposition bewirkt wird. Diese Reaktionsposition ist im Messraum innerhalb eines Löschabstandes (quenching distance) zu dem Gehäuse und/oder der Trennschicht ausgebildet. Als Löschabstand wird der Abstand zu Feststoffmassen (z.B. Wänden des Gehäuses) bezeichnet, innerhalb dessen keine Flammenbildung aufrechterhalten werden kann. Die Feststoffmassen nehmen Wärme auf und leiten diese auch ab und bewirken einen Abbruch der Reaktionskette. Der Löschabstand ist insbesondere abhängig vom Druck des Gemisches, der Temperatur der Wände des kühlenden Gehäuses und dem Anteilverhältnis von Brennstoff und Luft, und damit auch der Luftzahl. Zum Löschabstand wird hier auf die Fachliteratur verwiesen, z.B. "Verbrennung und Feuerungen", R. Günther, Springer-Verlag 2013. Der Löschabstand von einem stöchiometrischen Methan/Luft Gemisch bei Normaldruck beträgt z.B. etwa 2 mm und für Propan 1,8 mm.

Die Erfindung basiert auf der Erkenntnis, dass bei einer derartigen Ausbildung und Dimensionierung eines Sensors eine unkontrollierte Zündung mit darauffolgender Kettenreaktion in dem Messraum unterbunden wird. Gleichwohl zeigt sich, dass in unmittelbarer Umgebung des Energieeintrages, also an der Reaktionsposition, exotherme chemische Reaktionsprozesse in Gestalt einer Verbrennung provoziert werden können. Gemäß der Erfindung ist die Energiezufuhr in den Messraum steuerbar, da es sich um elektrisch betriebene Anregungsmittel handelt, welche in Abhängigkeit von der bereitgestellten elektrischen Energie unterschiedlich viel Energie in den Messraum einleiten. An der Position oder in dem Bereich des Messraumes, in dem Energie eingebracht wird, wird ein chemischer Prozess gestartet. Insbesondere werden dabei Vorstufen einer unkontrollierten Zündung benutzt. Es hat sich gezeigt, dass im Falle der Anregung von Kohlenwasserstoffen (beispielsweise Methan) durch die Anregungsmittel eine Radikalbildung erfolgt. Diese reaktionsfreudigen Komponenten, deren Anzahl von der Stärke der Anregung, also der Energiezufuhr abhängig ist, können dann in Abhängigkeit von der Luftzahl exotherm mit einem geringen Teil des im Messraum vorhandenen Sauerstoffs chemisch reagieren. In der Umgebung der Reaktionsposition wird also eine Art Zündungskeim gebildet, der jedoch angesichts der Dimensionierung und Lage der Reaktionsposition im Löschabstand zur Umgebung nicht zu einer unkontrollierten Ausbreitung der chemischen Reaktionsprozesse führen kann. Der Ablauf der chemischen Reaktionsprozesse wird vielmehr auf die Umgebung der Reaktionsposition beschränkt. Der Energieeintrag ist im Betrieb so zu regeln, dass räumlich eine stark beschränkte Reaktionsrate in Gang gesetzt wird. Die Reaktion ist eine Art kontrollierte Probenverbrennung und führt zu einer Emission von elektromagnetischer Strahlung im Zuge der exothermen Reaktionsprozesse bei Umsetzung mit einem Teil des Sauerstoffs im Messraum. Diese ausgesandte Strahlung wird gemäß der Erfindung durch eine optische Erfassungseinrichtung gemessen, deren Erfassungsbereich im Messraum auf die Reaktionsposition ausgerichtet ist. Die optische Erfassungseinrichtung überwacht die Intensität von Strahlung um wenigstens eine erste Wellenlänge herum und erzeugt aus der erfassten Strahlungsintensität mindestens ein Signal, aus welchem die Luftzahl ableitbar ist.

Die Erfindung nutzt den Effekt aus, dass die Emission von IR-, UV- und sichtbarem Licht (oder auch Strahlungserzeugung) der Reaktion bei Energieeintrag durch die Anregungsmittel in Abhängigkeit von der Luftzahl variiert. Die exothermen Reaktionsprozesse und auch die Ausdehnung des Reaktionsbereiches sind luftzahlabhängig. Dieser Effekt wird in dem erfindungsgemäßen Sensor ausgenutzt, um aus der Strahlungsintensität um wenigstens eine erste Wellenlänge herum die Luftzahl abzuleiten. Die Erfassung kann dabei in einem schmalen oder breiten Wellenlängenbereich um eine zentrale Wellenlänge herum erfolgen, beispielsweise in einem Bereich bekannter Anregungsniveaus beteiligter Moleküle, insbesondere im Bereich bekannter Anregungsniveaus von Wasser, welches bei der Verbrennung entsteht.

Die Intensität der ausgesandten Strahlung der Reaktion variiert mit der Luftzahl. Die Reaktionsrate ist höher, wenn die reaktionsfähigen Stoffe im passenden Mischungsverhältnis vorliegen. Dies kann z.B. der Fall sein, wenn die Luftzahl λ etwa gleich 1 ist. Bei dem optimalen Verhältnis bildet sich die Ausdehnung des Reaktionsbereiches maximal aus und/oder die Dichte der Reaktionsprozesse im Reaktionsbereich ist maximal. Damit ist dann auch die Strahlungsintensität um die Anregungsmittel herum maximal. Entsprechendes kann durch die optischen Erfassungseinrichtungen erkannt werden und genutzt werden, um die Luftzahl einzustellen, beispielsweise indem das Mischungsverhältnis variiert wird, bis das Intensitätssignal maximal ist. Dieser Punkt kann zu Kalibrierung des Sensors verwendet werden.

Das beschriebene Verfahren nutzt also einen Messraum mit Anregungsmitteln, die durch Eintrag von Energie chemische Reaktionsprozesse an der Reaktionsposition im Brenngas-Luft-Gemisch hervorrufen, ohne einen explosiven Reaktionsablauf zu ermöglichen. Die Strahlung dieser chemischen Reaktion wird durch eine optische Erfassungseinrichtung gemessen und die Strahlungsintensität wird zur Ermittlung der Luftzahl ausgewertet.

Die Reaktionsprodukte werden nach der Reaktion durch die gleiche Diffusionspassage im Messraum in den Strom des Brenngas-Luft-Gemisches diffundieren, durch welche Moleküle des Brennstoff-Luft-Gemisches zuvor eindiffundiert sind. Neues Brenngas-Luft-Gemisch diffundiert in Gegenrichtung nach. Der Messraum benötigt erfindungsgemäß entsprechend keinen Abgasanschluss, stattdessen werden die äußerst geringen Mengen von Verbrennungsprodukten durch die Diffusionspassage des Trennmittels in den Messgasstrom geleitet. Gleichwohl ist eine Ableitung von Gasen aus dem Messraum durch einen separaten Auslass grundsätzlich ebenfalls im Rahmen der Erfindung möglich.

Die Energiezufuhr wird in Abhängigkeit von dem gewählten Brenngas adaptiert. Eine zu starke Energiezufuhr führt nämlich gegebenenfalls zu chemischen Reaktionsprozessen in einem so großen Bereich, dass es zur Sättigung der chemischen Reaktion kommt. Während nämlich gemäß der Erfindung durch Steigerung der Energiezufuhr in dem Messraum zunächst die optisch gemessene Reaktion gestartet werden und die Strahlungsintensität des Reaktionsbereiches gesteigert wird, kann es bei einer solchen Steigerung dazu kommen, dass bei Erhöhung der Energiezufuhr wegen eines zu langsamen Wegdiffundierens der Abgase von der Reaktionsposition zur Sättigung des Reaktionsprozesses kommt. Für die eindiffundierenden Brenngas- und Sauerstoffmoleküle nimmt dann die Wahrscheinlichkeit einer Reaktion zwischen Radikalen des Brenngases und des Sauerstoffs ab, da in der Reaktionsposition noch Reaktionsprodukte vorliegen und den Zugang der Brenngas-Moleküle behindern. Entsprechend kann ein Arbeitspunkt gewählt werden, an dem die Energiezufuhr festgehalten wird und die Diffusionsprozesse einen kontinuierlichen Betrieb der Reaktion erlauben. Bei einem solchen Arbeitspunkt variiert bei konstanter Leistungszufuhr die Strahlungsintensität mit der Luftzahl und der erfindungsgemäße Sensor und das erfindungsgemäße Verfahren können zur Luftzahlbestimmung und Regelung eines Gemisches eingesetzt werden.

Der erfindungsgemäße Sensor kann alternativ auch in einem Regelbetrieb genutzt werden, bei dem die Strahlungsintensität der Reaktion durch Regelung konstant gehalten wird, indem die elektrische Leistung gesteuert wird. Die Strahlungsintensität wird dann in einem bestimmten Luftzahlbereich auf einem festen Sollwert gehalten und die erforderliche elektrische Leistung wird gemessen und zur Luftzahlbestimmung verwendet.

Gemäß einer ersten Alternative der Erfindung sind die Anregungsmittel als elektrische Heizeinrichtung mit einer ohmschen Heizung ausgebildet. Dabei ist ein stromdurchflossenes Heizelement vorgesehen, der innerhalb des Messraumes an der Reaktionsposition angeordnet ist. Die Anregungsmittel verfügen weiterhin über eine regelbare Strom- oder Spannungsquelle zur Versorgung dieses Heizelements.

Die Gestaltung der Sensoreinrichtung und die Durchführung des Verfahrens mit einer ohmschen Heizung sind ein äußerst kostengünstiger Weg der Konstruktion des Sensors. Außerdem gibt es bei Heizeinrichtungen mit ohmscher Heizung umfassenden Stand der Technik zur stabilen Gestaltung derartiger Heizeinrichtungen. Heizdrähte oder Heizspulen sind am Markt problemlos verfügbar. Beispielsweise können derartige Heizeinrichtungen aus hochtemperaturbeständigen Metalllegierungen gebildet sein, welche der geforderten thermischen Belastung über eine lange Zeitdauer gewachsen sind.

Die ohmsche Heizung wird beispielsweise über zwei oder auch mehrere Kontaktstellen in dem Messraum geführt und dort durch eine Strom- oder Spannungsregelung auf eine gewünschte Temperatur gebracht. Dabei kann die Abstrahlung der ohmschen Heizeinrichtung optisch überwacht werden, um die Temperatur nach dem Prinzip eines Pyrometers oder kontaktlosen Thermometers zu ermitteln und einzustellen. Die Wärme, welche das Heizelement bereitstellt, regt das umgebende Gasgemisch im Messraum an. Entsprechend ist in diesem Fall die Reaktionsposition die unmittelbare Umgebung entlang des Heizelements. Die Energiezufuhr erfolgt in diesem Fall also mittels Wärme. Es erfolgt eine Anregung des Gasgemisches, ein Start und die Aufrechterhaltung von Reaktionsprozessen der erzeugten Radikale in der unmittelbaren Umgebung des Heizelements.

Dabei ist vorzugsweise jedoch die optische Wirkung des beheizten Heizelements in ihrer Strahlung im durch Filterung selektierten Wellenlängenbereich deutlich verschoben gegenüber der Wellenlänge, die mit der optischen Erfassungseinrichtung zur Ermittlung der Luftzahl abgeleitet wird.

Alternativ können zwei optische Erfassungseinrichtungen eingesetzt werden, wobei eine davon selektiv für die Strahlung der überwachten Reaktion ist und die zweite optische Erfassungseinrichtung neben dem Intensitätssignal der überwachten Reaktion auch die sichtbare Lichtausstrahlung des Heizelements misst. Das Differenzsignal der Erfassungseinrichtungen wird dann für die Berechnung der Temperatur des Heizelements angewandt. Entsprechend lässt sich die Ausstrahlung durch das Heizelement verlässlich messen.

Die optische Erfassungseinrichtung kann mit Filtern zur Selektion der zu überwachenden Wellenlängen ausgestattet sein. Die Erfassungseinrichtung für die Reaktionsausstrahlung erfasst beispielsweise im Wellenlängenbereich von 900 nm bis 1100 nm eine Strahlungsemission eines Anregungsniveaus von Wasser. Gleichzeitig wird auch ein störender Einfluss der IR-Strahlung des Heizelements erfasst. Der Einfluss dieser IR-Strahlung kann mittels Nutzung des Signals der erfassten Strahlung des Heizelements im sichtbaren Spektrum kompensiert werden, wodurch die Reaktionsausstrahlung nahezu unabhängig von der infraroten Ausstrahlung des Heizelements gemessen wird.

Ein entsprechend aufgebauter Sensor ist günstig in der Herstellung, lange haltbar und unkritisch hinsichtlich anliegender Spannungen.

Gemäß einer alternativen Ausführungsform der Erfindung weisen die Anregungsmittel mehrere konstruktiv und galvanisch getrennte Elektroden auf. Diese ragen wenigstens teilweise in den Messraum hinein und das Anregungsmittel hat eine Spannungsversorgung, welche eine Spannung, insbesondere auch eine Hochspannung an die Elektroden anlegen kann. Diese Ausführungsform dient dazu, ein energiereiches elektrisches Feld zwischen den Elektroden aufzubauen, so dass dort Reaktionen begünstigt werden. In einer Abwandlung dieser Ausführungsform können durch Hochspannungspulse Zündfunken erzeugt werden, die eine entsprechende Energiezufuhr in diesem Bereich erlauben.

In einer bevorzugten Ausführungsform ist eine Messeinrichtung zur Erfassung der elektrischen Leistung durch das Anregungsmittel in dem Messraum ausgebildet. Mit einer solchen Messeinrichtung besteht jederzeit die Kontrolle und Möglichkeit der Regelung der zugeführten Leistung.

Alternativ kann eine Überwachung der Anregungsmittel durch unabhängige Messeinrichtungen, insbesondere optische Messeinrichtungen, zum Bespiel in Gestalt kontaktloser Temperaturmessgeräte verwendet werden.

Die gemessenen Werte einer Leistungszufuhr können zur Steuerung und Kalibrierung der Messeinrichtungsteile verwendet werden. Insbesondere können zur Aufnahme einer Kalibrierkurve die eingebrachte Leistung mit dem optischen Intensitätssignal der überwachten Reaktion gemeinsam aufgenommen werden, um einen optimalen Arbeitspunkt zu finden.

In einer nicht von den Ansprüchen umfassten Ausführungsform ist als Bestandteil der Anregungsmittel eine Laserquelle vorgesehen, welche auf die Reaktionsposition abstrahlt. Hoch intensives Laserlicht einer geeigneten Wellenlänge (zum Beispiel UV-Strahlung), die insbesondere auf ein Anregungsniveau eines Brennstoffes selektiert ist, kann zur Energiezufuhr zur Reaktionsposition dienen.

In einer besonders bevorzugten und einfachen Gestaltung ist die optische Erfassungseinrichtung mit einem optisch sensitiven Mittel in Gestalt einer Fotodiode, eines Fototransistors, eines Fotowiderstandes oder auch eines Fotodiodenarrays ausgebildet. Sämtliche dieser Bauteile sind in marktüblicher Weise verfügbar und einsetzbar.

Es ist bevorzugt, wenn die optisch sensitiven Mittel mit optischen Filtern zur Selektion eines Wellenlängenbereiches versehen sind.

Wie oben bereits beschrieben, kann von den Anregungsmitteln selbst Strahlung erzeugt werden, die grundsätzlich auch von den optischen Erfassungseinrichtungen messbar ist. Dies gilt insbesondere bei infraroter und sichtbarer Lichtausstrahlung von Heizelementen, ebenso jedoch auch bei Hochspannungsblitzen oder bei Laserpulsen. Die Einschränkung des detektierten Wellenlängenspektrums durch ein optisches Filter an der optischen Erfassungseinrichtung liefert einen besseren Signalrauschabstand und unterdrückt die Detektion von Strahlung, welche nicht durch chemische Reaktionsprozesse hervorgerufen wird. Über den Vergleich der Signale von zwei oder mehreren optischen Erfassungseinrichtungen mit unterschiedlichen Wellenlängensensitivitäten können die Strahlungsintensität der chemischen Reaktion und die des Heizelements getrennt werden.

Es ist weiterhin bevorzugt, dass das gasdurchlässige Trennmittel, welches die Diffusionspassage bildet, als gesinterte Scheibe oder als Gitter ausgebildet ist.

Das Trennmittel hat außer der Wirkung als Diffusionspassage auch eine Wirkung als Flammschutz, außerdem vermeidet es die Übertragung von Turbulenzen in den Messraum und das Eindringen von Fremdkörpern. Das Trennmittel ist aus hitzebeständigem und nicht-brennbarem Material zu fertigen.

Es ist weiterhin bevorzugt, wenn die Anregungsmittel mit einer Regelung gekoppelt sind, um die Energiezufuhr in den Messraum zu regeln. Dabei kann die Regelung in Abhängigkeit von dem Signal der optischen Erfassungseinrichtung erfolgen.

Die durch die optische Erfassungseinrichtung detektierte Strahlungsintensität kann auf die Regelung der Energiezufuhr durch das Anregungsmittel zurückwirken. Dies ist insbesondere dann sinnvoll, wenn keine oder nur eine geringe Strahlungsintensität detektiert wird, also keine detektierbare chemische Reaktion stattfindet. In diesem Fall kann die Leistungszufuhr über die Anregungsmittel erhöht werden. Außerdem kann in Abhängigkeit von der gemessenen Strahlungsintensität ein optimaler Arbeitspunkt angefahren werden. Es hat sich gezeigt, dass beim Starten des Sensors und einer Erhöhung der Leistungszufuhr zunächst eine exotherme chemische Reaktion in einem kleinen Bereich gestartet wird, wobei sich dieser Bereich und die chemische Reaktionsrate bei weiterer Erhöhung der Leistungszufuhr vergrößern. In Abhängigkeit des verfügbaren Brennstoff-Luft-Gemisches und der Größe des Messraumes sowie den weiteren konstruktiven Eigenschaften des Sensors, geschieht die Vergrößerung des Brennbereiches jedoch nur bis zu einer Grenze, bei deren Überschreitung die Geschwindigkeit des Stofftransports von Brennstoff zu den Anregungsmitteln und der Abtransport von Reaktionsprodukten über die Diffusionspassage eine bestimmende Größe wird. In diesem Bereich führt eine Erhöhung der Leistungszufuhr nicht länger zu einer Zunahme der chemischen Reaktionsrate, es kann vielmehr zu einem teilweisen oder völligen Zusammenbruch der Reaktion und einem oszillierenden Reaktionsprozess oder einem pulsierenden, wiederholten Neustart der Reaktionsprozesse nach einem teilweisen Ausgleich der Stoffverhältnisse durch Diffusionsvorgänge kommen. Die Konstruktion des Sensors kann auch so gestaltet werden, dass der Stoffaustausch über die Diffusionspassage so groß ist, dass das Heizelement wegen seiner beschränkten Größe, auch bei erhöhten Temperaturen, nicht mehr Brenngas-Luft-Gemisch mittels chemischer Reaktionen umsetzen kann als über die Diffusionspassage zugeliefert werden kann. Bei so einer Konstruktion des Sensors kann es, weil die Reaktionsprodukte nicht ausreichend schnell aus der Reaktionsposition abgeführt werden können und deshalb der Nachschub von Brenngas- und Sauerstoffmoleküle begrenzt wird, zur Sättigung der chemischen Reaktion in der Reaktionsposition kommen.

Falls eine kontinuierliche Reaktion erwünscht wird, kann in Abhängigkeit von dem gemessenen optischen Signal eine Einstellung der Leistungszufuhr an dem Anregungsmittel vorgenommen werden.

Es ist in einer weiteren Ausführungsform der Erfindung bevorzugt, wenn die Messeinrichtung zur Erfassung der Leistungszufuhr über einen optischen Sensor verfügt, welcher das Anregungsmittel im Messraum überwacht.

Wie bereits vorstehend beschrieben, kann beispielsweise eine Heizeinrichtung nach Prinzip eines Pyrometers überwacht werden. Dies kann auch mit einer Fotodiode geschehen, welche die in einem spektralen Bereich gefilterte Ausstrahlung eines erhitzten Glühdrahtes oder Heizelements misst. Aus dem Signal der Fotodiode kann ein Maß für die aktuelle Temperatur abgeleitet werden.

Das erfindungsgemäße Verfahren nutzt bevorzugt die beschriebene Sensoreinrichtung. Es weist in jedem Fall den Schritt auf, einen Messraum mit einem zu überwachenden Brenngas-Luft-Gemisch-Strom zu koppeln, wobei zwischen Messraum und Gasstrom das gasdurchlässige Trennmittel, welches die Diffusionspassage bildet, ausgebildet wird. Das im Messraum befindliche Brenngas-Luft-Gemisch wird dann mit dem elektrisch betriebenen Anregungsmittel stimuliert, um gezielt chemische Reaktionsprozesse des aus dem Gasstrom hineindiffundierten Brenngas-Luft-Gemisches herbeizuführen. Diese chemische Reaktion wird optisch gemessen, wobei die optische Erfassungseinrichtung in dem Messraum auf mindestens ein Bereich der Reaktionsposition gerichtet ist und mindestens einen spektralen Bereich der Anregungsniveaus wenigstens eines Reaktionsproduktes misst. Dieser spektrale Bereich umfasst zum Beispiel als zentrale Wellenlänge einen erwarteten Abstrahlungsübergang der Reaktionsprodukte. Insbesondere können hier exotherme chemische Reaktionen die durch Schwingungs- und Rotationsniveaus des Wassermoleküls im Reaktionsbereich zur Lichtausstrahlung führen, gemessen werden. Beispielsweise bietet sich an, den Wellenlängenbereich von etwa 900 nm bis 1100 nm zu überwachen, welcher charakteristisch für einen Anregungsmodus des Wassermoleküls ist.

Die chemische Reaktion wird in dem Messraum so durchgeführt, dass es zu keiner Zeit zu einer explosiven oder unkontrollierten exothermen chemischen Reaktion kommen kann. Dies wird dadurch erreicht, dass die Anregungsenergie für die provozierte chemische Reaktion an einer Stelle in dem Messraum eingebracht wird, die innerhalb des Löschabstandes zur Umgebung, also insbesondere zu den Gehäusewandungen angeordnet ist. Auf diese Weise wird nur in unmittelbarer Nähe des Anregungsmittels die Energie dem Messraum zugeführt, beispielsweise durch ein Heizelement in der Form eines geraden oder geformten Heizdrahts. Es findet also eine exotherme chemische Reaktion statt, wobei es jedoch nicht zu einer größeren Flammenbildung kommt, sondern Reaktionsprozesse der erzeugte Radikale innerhalb des Löschabstandes durch kontinuierliche externe Energiezufuhr aufrechterhalten werden. Würde die zugeführte Energie abgestellt, würde die Reaktion sofort enden, da der Reaktionserhalt auf die externe Energiezufuhr angewiesen ist. Außerdem bildet der Heizdraht wegen seiner Temperatur eine Flammsperre deren Löschabstand sich bei Temperatursenkung vergrößert. Gleichwohl ist aus der Reaktion die Luftzahl ableitbar.

Die Erfindung wird nun anhand der beiliegenden Figur näher erläutert.
Figur 1 zeigt den schematischen Aufbau einer Ausführungsform der Erfindung;
Figur 2 zeigt einen beispielhaften Strahlungsintensitätsverlauf in Abhängigkeit von der Luftzahl.

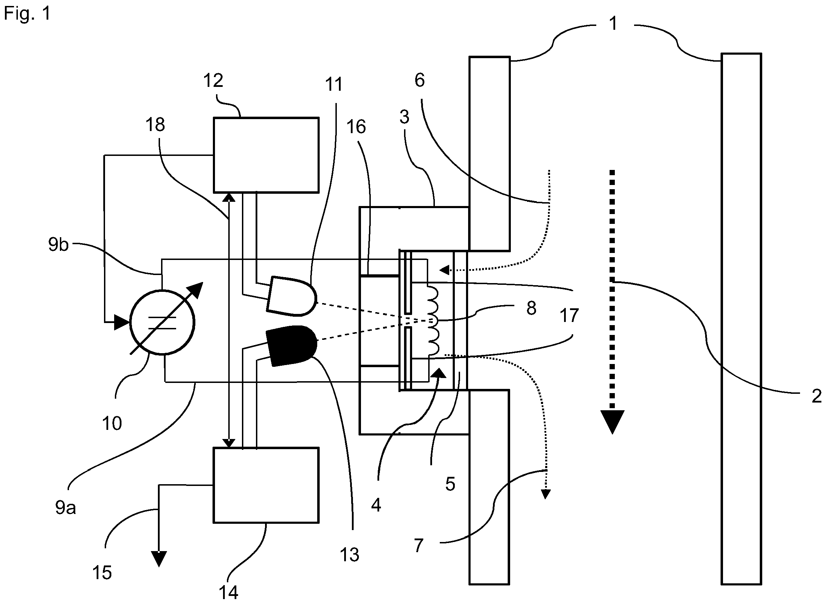

In Figur 1 ist eine Zuleitung 1 für ein Brenngas-Luft-Gemisch gezeigt. Entlang dieser Zuleitung wird in Richtung des Pfeiles 2 das Brenngas-Luft-Gemisch zu einem Verbraucher, beispielsweise einem Heizbrenner zugeführt. In einer Öffnung der Leitung 1 ist ein Sensorgehäuse 3 gasdicht mit der Leitung 1 gekoppelt. Das Sensorgehäuse 3 weist einen Hohlraum 4 auf, der durch eine gesinterte Scheibe 5 von dem Gasstrom 2 konstruktiv getrennt ist. Die gesinterte Scheibe 5 dient als Trennmittel dem Flammschutz und zur Unterdrückung möglicher Turbulenz. Die gesinterte Scheibe 5 ist für das Gas durchlässig, so dass gemäß Pfeil 6 jederzeit aus dem Gasstrom 2 Anteile des Brenngas-Luft-Gemisches in dem Messraum 4 eindiffundieren können. Auf derselben Weise können die Stoffe aus dem Raum 4 entlang der Richtung des Pfeiles 7 in den Gasstrom 2 gelangen.

In dem Messraum 4 ist ein Heizdraht 8 in Gestalt einer Heizwendel angeordnet. Die Leitungszuführungen 9a, 9b zu dem Heizdraht 8 sind durch das Gehäuse 3 gasdicht hindurchgeführt und mit einer regelbaren Spannungsquelle 10 gekoppelt. In Abhängigkeit von der eingestellten Spannung an der Spannungsquelle 10 wird der Heizdraht 8 aufgeheizt.

In diesem Ausführungsbeispiel sind zwei Fotodioden 11, 13 außerhalb des Messraums angeordnet. Der Messraum weist auf seiner, den Fotodioden 11, 13 zugewandten, Seite ein Fenster 16 auf, durch welches Strahlung aus dem Messraum 4 zu den Fotodioden 11, 13 gelangt. Zwischen dem Fenster 16 und dem Heizdraht 8 ist eine Blende 17 angeordnet, welche den Sichtbereich der Fotodioden 11, 13 auf einen Abschnitt des Heizdrahtes 8 beschränkt.

Die Fotodiode 11 ist mit ihrer spektralen Sensitivität auf den Wellenlängenbereich des sichtbaren Lichts eingestellt. Damit misst sie die Lichtausstrahlung des obengenannten Spektrums der Heizwendel 8. Die Fotodiode 11 ist mit einer Auswerteschaltung 12 gekoppelt, welche die Signale der Fotodiode verarbeitet. Über die Messung der emittierten Strahlung wird ein Maß für die Temperatur der Heizwendel 8 ermittelt und die Auswerteschaltung 12 kann die Spannungsquelle 10 ansteuern, um der gemessenen Maß für die Ist-Temperatur, ermittelt aus den Signalen der Fotodiode 11, dem vorgegebenen Sollwert anzunähern.

Mit der Fotodiode 13 ist eine weitere Auswerteschaltung 14 gekoppelt. Die Fotodiode 13 ist in diesem Ausführungsbeispiel ausgewählt, um elektromagnetische Strahlung von Reaktionsprodukten im Bereich der Heizspule 8 zu erfassen. Es handelt sich in diesem Ausführungsbeispiel um eine Fotodiode, deren Empfindlichkeitsbereich in dem Wellenlängenbereich von 800 nm bis 1100 nm besonders ausgeprägt ist.

Die Auswerteschaltung 12 und die Auswerteschaltung 14 sind über die Verbindung 18 gekoppelt. Über diese Verbindung können Daten zwischen den Auswerteschaltungen ausgetauscht werden (in der Praxis können die Auswerteschaltungen 12 und 14 auch zu einer Schaltung zusammengefasst sein).

Das Signal dieser Fotodiode 13 wird von der Auswerteschaltung 14 gewandelt, um ein Messsignal 15 auszugeben. Dieses Messsignal 15 wird in diesem Ausführungsbeispiel verwendet, um die aktuelle Luftzahl anhand von Kalibrierungsdaten zu berechnen.

Mit der gezeigten Ausführungsform der Erfindung ist eine rasche und verlässliche Ermittlung der Luftzahl des vorbeiströmenden Brenngas-Luft-Gemisches 2 möglich. Wird ein Brenngas-Luft-Gemisch entlang der Pfeilrichtung 2 an dem Messraum 4 vorbeigeleitet, diffundiert jederzeit durch die Diffusionsprozesse ein Teil dieses Brenngas-Luft-Gemisches in dem Messraum 4 ein. Dort gelangt ein kleiner Anteil der Moleküle des Brenngas-Luft-Gemisches in Kontakt mit dem Heizdraht 8. Ein wesentlicher Aspekt der Erfindung besteht darin, dass in dem Messraum 4 unter kontrollierten und sicheren Bedingungen die exotherme chemische Reaktion eines kleinen Anteils des Brenngas-Luft-Gemisches erfolgt. Da sich der Heizdraht 8 innerhalb des Löschabstandes von den umgebenden Wandungen befindet und außerdem die gesinterte Scheibe 5 einen Flammschutz bildet, ist diese kontrollierte exotherme chemische Reaktion jederzeit sicher. Die exotherme chemische Reaktion in unmittelbarer Umgebung des Heizdrahtes 8 wird nur aufrechterhalten, da kontinuierlich Energie über die Spannungsquelle 10 zugeführt wird. Eine selbsterhaltende Verbrennung in dem Messraum 4 ist aufgrund der Anordnung des Heizdrahtes im Löschabstand nicht möglich. Dass es dennoch zu einer stimulierten exothermen chemischen Reaktion in unmittelbarer Umgebung des Heizdrahtes 8 kommt, liegt an der kontinuierlich zugeführten Energie. Auf diese Weise wird die optische Messung der chemischen Reaktion und insbesondere die Erfassung der Intensität der ausgesandten Strahlung ermöglicht. Dabei ist die optische Erfassung über die Fotodiode 13, durch einen optischen Filter so auf die chemische Reaktion angepasst, dass charakteristische Anregungsniveaus der Reaktionsprodukte bei den chemischen Reaktionsprozessen gemessen werden. In diesem Ausführungsbeispiel wird eine Emission einer Vibrations-Rotations-Anregung von Wasser im Bereich um 1000 nm als charakteristische Wellenlänge gemessen. Entsprechend ist die spektrale Empfindlichkeit der Fotodiode 13 gewählt.

Der erfindungsgemäße Sensor und das erfindungsgemäße Verfahren bezwecken also eine exotherme chemische Probereaktion des vorbeiströmenden Brenngas-Luft-Gemisches, wobei diese exotherme chemische Probereaktion nicht zur Zündung des Brenngas-Luft-Gemisches des Versorgungsgasstroms 2 führen kann.

Die Beobachtungen bei Messungen mit dem Aufbau des genannten Ausführungsbeispiels zeigen bei einem Brenngas, welches hauptsächlich aus Methan besteht, dass beim Start der Einrichtung und bei einem anfänglichen Aufheizen der Heizspule 8 zunächst Wärme über das Gasgemisch an die Wandungen 3 abgeführt wird. Bei einer Steigerung der Heiztemperatur des Heizdrahtes 8, überwacht von der Fotodiode 11, kommt es zu exothermen chemischen Reaktionsprozessen an der Oberfläche des Heizdrahtes 8. Dies wird zumindest durch die Fotodiode 13 gemessen. Dabei bildet sich zunächst eine dünne Reaktionsschicht über dem Heizdraht 8, dessen Lichtintensität im überwachten Wellenlängenbereich um 1000 nm mit Temperatursteigerung weiter ansteigt. Die Dicke der Reaktionsschicht und die Dichte der ablaufenden Reaktionsprozesse in der Reaktionsposition um den Heizdraht 8 nehmen dabei mit steigender Temperatur zu.

Falls der Stofftransport von Brenngas- und Sauerstoffmolekülen über die Diffusionspassage geringer ist als die Rate der am Heizdraht reagierenden Moleküle kommt es bei weiterer Steigerung der Temperatur schließlich zu einem weitgehenden Reaktionszusammenbruch und (luftzahlabhängig) zu einem anschließenden Neuanstieg der Strahlungsintensität. Es kommt also zu einer Art Pulsieren der Lichtintensität. Falls über die Diffusionspassage jedoch ausreichend Brenngas- und Sauerstoffmoleküle für die exothermen chemischen Reaktionsprozesse zugeführt werden, werden bei weiter steigender Temperatur solche Mengen an Reaktionsprodukten freigesetzt, dass diese den Heizdraht umlagern und den Zustrom von Brenngas- und Sauerstoffmolekülen begrenzen. Es kommt zu einer Sättigung der exothermen chemischen Reaktionen. Dies führt dann dazu, dass auch die Strahlungsintensität der chemischen Reaktion gesättigt ist.

Entsprechend wird die Temperatur des Heizdrahts 8 so gewählt, dass eine kontinuierliche, nicht oszillierende oder nicht gesättigte chemische Reaktion an der Oberfläche des Heizdrahts 8 beobachtet wird. Dabei kann zum Beispiel zunächst ein oszillierender oder gesättigter Betrieb angefahren werden und dann die Temperatur verringert werden. Die Auswertung der Ausstrahlung im nicht-oszillierenden Betrieb ist in diesem Beispiel die bevorzugte Variante, es kann grundsätzlich jedoch auch der oszillierende Betrieb für eine Auswertung verwendet werden.

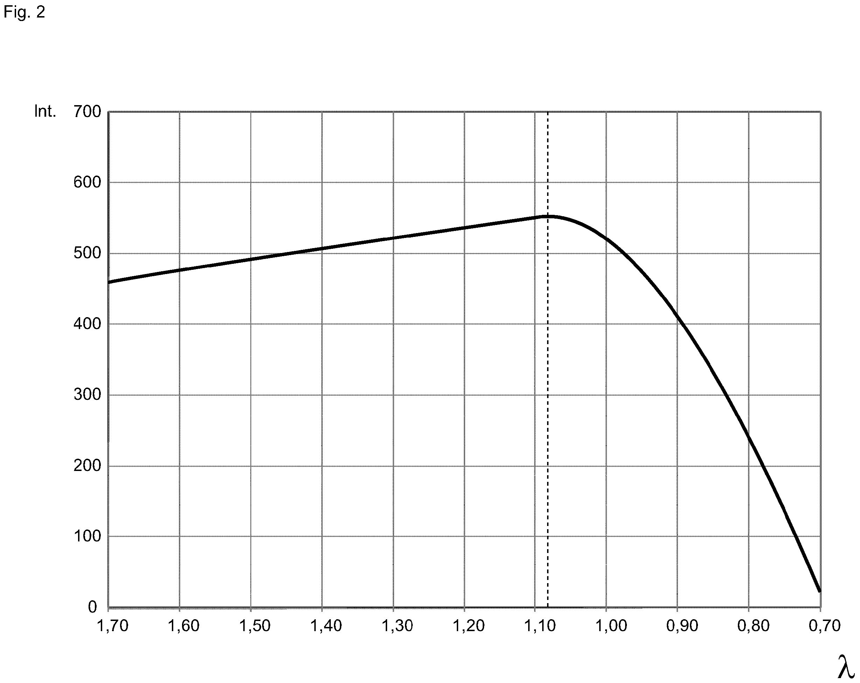

In Figur 2 ist ein Messverlauf der erfindungsgemäßen Sensoreinrichtung gemäß dem Ausführungsbeispiel gezeigt. Die Daten sind aus gemittelten Messdaten erzeugt und erstrecken sich über einen Messbereich der Luftzahl von A=0,70 bis λ=1,70. Die Luftzahl wurde dabei im Gasstrom durch ein herkömmliches Gasanalysegerät ermittelt. Die Skalierung der Intensitätsachse (Y-Achse) ist für eine qualitative Darstellung der Messwerte gewählt worden.

Es zeigt sich der oben beschriebene Kurvenverlauf mit einem nahezu linearen Verlauf für magere Gemische und einem steilen Abfall hin zu zunehmend fetten Gemischen.

Das lineare Ansteigen der Intensität bei Luftzahländerung von 1,70 nach 1,10 kann dadurch erklärt werden, dass es immer weniger Luftüberschuss gibt und deshalb die Temperatur der Reaktionsschicht um den Heizdraht steigt. Dies sorgt dafür, dass mehr Radikale gebildet werden und die Reaktionsschicht um den Heizdraht sich ausdehnt und/oder die die Dichte der wasserformenden, exothermen chemischen Reaktionsprozesse zunimmt, was zu einer höheren Lichtausstrahlung im Wellenlängenbereich um 1000 nm führt.

Das Maximum der Kurve liegt bei der Anwendung von Methan als Brenngas nahe dem stöchiometrischen Gemisch, jedoch nicht genau bei λ=1 sondern etwa bei λ=1,07. Bei einem Propan-Luft-Gemisch verschiebt sich die Kurve nach rechts. Das Maximum liegt dann (in Abhängigkeit der Heizdrahttemperatur) bei etwa λ=0,9.

Um eine Kalibrierung auf λ=1,0 vorzunehmen, bietet der erfindungsgemäße Sensor eine weitere Einsatzmöglichkeit.

Bei einer gezielten Reduzierung der Temperatur des Heizdrahtes wird der Wirkungsbereich des Sensors nämlich eingeschränkt. Vor allem im Bereich λ<=1 brechen die detektierbaren Reaktionsprozesse zusammen. Dies erlaubt es durch Zusammenspiel mit der Brennersteuerung und einer Variation des Brenngas-Luft-Verhältnisses ein Brenngas-Luft-Gemisch bereitzustellen, welches gerade noch Reaktionsprozesse erlaubt.

Es wird also z.B. iterativ sowohl das Gemischverhältnis variiert als auch die Temperatur des Heizdrahtes reduziert, bis ein Punkt aufgefunden wird, der noch einen Reaktionsbetrieb mit Strahlungsemission erlaubt, von dem aus aber in Richtung eines mageren und fetteren Gemisches jeweils ein Zusammenbruch der Reaktion (Verschwinden der Strahlung) erfolgt.

Das so aufgefundene Brenngas-Luft-Gemisch hat, unabhängig von der Art des Brenngases, eine Luftzahl von etwa λ=1,0.

Nachdem dieser Punkt aufgefunden wurde, wird unter Beibehaltung des so eingeregelten Brenngas-Luft-Verhältnisses der Heizdraht wieder auf eine normale Betriebstemperatur hochgeregelt. Die Größe des Signals entspricht dann dem Wert für eine Luftzahl von λ=1,0.

Es gibt also zwei Punkte, die charakteristisch für den Brennerbetrieb anhand der Messungen aufgefunden werden können. Der Punkt des oben genannten Maximums der Strahlungsintensität stellt gasartabhängig einen Fixpunkt dar. Außerdem kann der Sensor auf den Punkt λ=1,0 kalibriert werden.

Das erfindungsgemäße Verfahren und auch der erfindungsgemäße Sensor sind in vielerlei Hinsicht abzuwandeln. Beispielsweise kann der Sensor mehrteilig konstruiert sein und grundsätzlich ist es auch möglich, am Sensor eine Gasabführung vorzusehen, statt das Gas in den Gasstrom zu leiten. Wesentlich ist, dass ein Teil des Gasstromes abgezweigt wird und dass die emittierte Ausstrahlung einer kontrollierten exothermen chemischen Reaktion optisch gemessen wird, um die Luftzahl zu bestimmen. Daraufhin kann eine Optimierung des Brenngas-Luft-Gemisches vorgenommen werden. Den bekannten Verfahren aus dem Stand der Technik, bei denen im Wesentlichen elektrochemische Effekte zur Luftzahlbestimmung genutzt werden, wird also eine Alternative mit einer optischen Messung einer chemischen Probereaktion an die Seite gestellt. Die chemische Probereaktion findet dabei jederzeit kontrolliert statt und in einem derart dimensionierten Aufbau, dass keine selbsterhaltende oder unkontrollierte exotherme chemische Reaktion des Brenngas-Luft-Gemisches im Messraum möglich ist.

## Patentansprüche

1. Sensor zur Bestimmung einer Luftzahl eines Brenngas-Luft-Gemisches (2),
mit einem Gehäuse (3) welches einen Messraum (4) begrenzt,
wobei das Gehäuse (3) wenigstens auf einer Seite eine Diffusionspassage zur Kopplung mit einem Brenngas-Luft-Gemisch-Strom (2) aufweist, wobei die Diffusionspassage durch ein gasdurchlässiges Trennmittel (5) gebildet ist,
wobei ein elektrisch betriebenes Anregungsmittel (8) für eine Energiezufuhr in den Messraum (4) umfasst ist, um eine chemische Reaktion eines Brenngas-Luft-Gemisches in dem Messraum herbeizuführen, wobei das Anregungsmittel (8) ausgebildet und ausgerichtet ist, um die Energiezufuhr an einer im Messraum befindlichen Reaktionsposition zu bewirken, die innerhalb des einem zu verwendenden Brenngas zugeordneten Löschabstandes zu dem Gehäuse (3) oder dem Trennmittel (5) angeordnet ist, **dadurch gekennzeichnet, dass** das Anregungsmittel (8)
- als elektrische Heizeinrichtung mit einem stromdurchflossenen ohmschen Heizelement ausgebildet ist und wobei das Heizelement innerhalb des Messraums an der Reaktionsposition angeordnet ist,
oder dass das Anregungsmittel (8)
- mit mehreren, konstruktiv und galvanisch getrennten Elektroden ausgebildet ist, welche wenigstens teilweise in den Messraum hineinragen, wobei die Reaktionsposition zwischen den Elektroden liegt,
wobei das Anregungsmittel mit einer Spannungsversorgung gekoppelt ist,
wobei wenigstens eine optische Erfassungseinrichtung (13, 14) umfasst ist, deren Erfassungsbereich in dem Messraum (4) auf die Reaktionsposition ausgerichtet ist, wobei die optische Erfassungseinrichtung (13, 14) die Intensität von Strahlung aus der Reaktionsposition in wenigstens einem ersten Wellenlängenbereich erfasst und ein der erfassten Intensität zugeordnetes Signal (15) erzeugt, aus welchem die Luftzahl ableitbar ist.

2. Sensor nach Anspruch 1, wobei eine Messeinrichtung (11, 12) zur quantitativen Erfassung der Energiezufuhr durch das Anregungsmittel ausgebildet ist.

3. Sensor nach einem der vorangehenden Ansprüche, wobei die optische Erfassungseinrichtung ein optisch sensitives Mittel aufweist.

4. Sensor nach Anspruch 3, wobei das optisch sensitive Mittel mit einem optischen Filter zur Selektion eines Wellenlängenbereichs versehen ist.

5. Sensor nach einem der vorangehenden Ansprüche, wobei ein optisches Selektionsmittel zwischen der Reaktionsposition und der optischen Erfassungseinrichtung angeordnet ist, um die Ausstrahlung aus einem Teilbereich der Reaktionsposition zur Erfassung durch die optische Einrichtung zu selektieren.

6. Sensor nach Anspruch 5, wobei das optische Selektionsmittel als Blende oder als Lichtleiter oder als Linse ausgebildet ist.

7. Sensor nach einem der vorangehenden Ansprüche, wobei eine Regelung mit den Anregungsmitteln gekoppelt ist, um den Energiezufuhr in den Messraum zu regeln, wobei die Regelung wenigstens teilweise in Abhängigkeit von dem Signal der optischen Erfassungseinrichtung erfolgt.

8. Sensor nach einem der Ansprüche 2 bis 7, wobei die Messeinrichtung zur Erfassung der Energiezufuhr mit wenigstens einem optischen Sensor ausgebildet ist, welcher das Anregungsmittel im Messraum optisch überwacht.

9. Verfahren zum Bestimmen der Luftzahl eines Brenngas-Luft-Gemisches, mit den Schritten
Koppeln eines Messraums mit einem zu überwachenden Gasstrom, wobei der Messraum durch ein gasdurchlässiges Trennmittel, welches eine Diffusionspassage bildet, mit dem Gasstrom gekoppelt wird,
Anregen des im Messraum befindlichen Gases mit einem elektrisch betriebenen Anregungsmittel an einer Reaktionsposition, um eine chemische Reaktion eines Brenngas-Luft-Gemisches in dem Messraum herbeizuführen,
wobei die Reaktionsposition innerhalb des einem zu verwendenden Brenngas zugeordneten Löschabstandes zu dem Gehäuse oder dem Trennmittel angeordnet ist,
wobei als Anregungsmittel (8) verwendet wird:
- eine elektrische Heizeinrichtung mit einem stromdurchflossenen ohmschen Heizelement, wobei das Heizelement innerhalb des Messraums an der Reaktionsposition angeordnet ist,
oder
- mehrere, konstruktiv und galvanisch getrennte Elektroden, welche wenigstens teilweise in den Messraum hineinragen, wobei die Reaktionsposition zwischen den Elektroden liegt
wobei das Anregungsmittel mit einer Spannungsversorgung gekoppelt ist,
Überwachen des Messraums mit einer optischen Erfassungseinrichtung, deren Erfassungsbereich auf die Reaktionsposition gerichtet ist, in einem spektralen Bereich um wenigstens eine erste Wellenlänge, wobei die Intensität der Ausstrahlung ermittelt wird,
Berechnen der Luftzahl aus den ermittelten Strahlungsintensitäten.

10. Verfahren nach Anspruch 9, wobei die Luftzahl anhand einer tabellarischen Zuordnung aus der gemessenen Intensität ermittelt wird.

11. Verfahren nach Anspruch 9, wobei die elektrische Heizung so geregelt wird, dass die von der optischen Erfassungseinrichtung gemessene Lichtausstrahlung der chemischen Reaktionsprozesse auf einem Sollwert gehalten wird.

12. Verfahren nach einem der Ansprüche 9 bis 11, wobei das Brenngas-Luft-Verhältnis iterativ verändert wird und die Energiezufuhr zum Anregungsmittel so lange verringert wird, bis ein Punkt aufgefunden wird, der noch einen Reaktionsbetrieb mit Strahlungsemission erlaubt, von dem aus aber in Richtung eines mageren und fetteren Gemisches jeweils ein Zusammenbruch der Reaktion erfolgt, um diesem Punkt eine Luftzahl des Brenngas-Luft-Gemisches von λ=1,0 zuzuweisen.

## Claims

1. Sensor for determining an air ratio of a combustible gas-air mixture (2),
with a housing (3) which limits a measuring space (4),
wherein the housing (3) at least on one side has a diffusion passage for coupling with a combustible gas-air mixture flow (2), wherein the diffusion passage is formed by a gas permeable separation means (5),
wherein an electrically operated stimulating means (8) for an energy supply into the measuring space (4) is included, in order to bring about a chemical reaction of a combustible gas-air mixture in the measuring space,
wherein
the stimulating means (8) is designed and adapted to effect the energy supply at a reaction position situated in the measuring space, which is disposed within the quenching distance, belonging to one combustible gas which is to be used, to the housing (3) or the separation means (5),
**characterised in that** the stimulating means (8)
- is formed as electrical heating device with a resistive heating element through which electrical current flows and wherein the heating element is disposed within the measuring space at the reaction position,
or that the stimulating means (8)
- is formed with a plurality of constructively and galvanically separated electrodes which protrude at least partially into the measuring space, wherein the reaction position lies between the electrodes,
wherein the stimulating means is coupled with a voltage supply,
wherein at least one optical detecting device (13, 14) is included, the detection area of which is directed in the measuring space (4) onto the reaction position, wherein the optical detecting device (13, 14) detects the intensity of radiation from the reaction position in at least one wavelength range and generates a signal (15) assigned to the detected intensity, from which the air ratio can be calculated.

2. Sensor according to claim 1, wherein a measuring device (11, 12) is formed for the quantitative detection of the energy supply by the stimulating means.

3. Sensor according to one of the preceding claims, wherein the optical detecting device has an optically sensitive means.

4. Sensor according to claim 3, wherein the optically sensitive means has an optical filter for selecting a wavelength range.

5. Sensor according to one of the preceding claims, wherein an optical selection means is disposed between the reaction position and the optical detecting device, in order to select the radiation out of a partial area of the reaction position for detecting using the optical device.

6. Sensor according to claim 5, wherein the optical selection means is formed as aperture or as light guide or as lens.

7. Sensor according to one of the preceding claims, wherein an adjustment control is coupled with the stimulating means in order to adjust the energy supply into the measuring space, wherein the adjustment control is carried out at least partially depending on the signal of the optical detecting device.

8. Sensor according to one of claims 2 to 7, wherein the measuring device for detecting the energy supply is formed with at least one optical sensor which optically monitors the stimulating means in the measuring space.

9. Method for determining the air ratio of a combustible gas-air mixture, with the steps
coupling a measuring space with a gas flow which is to be monitored, wherein the measuring space is coupled with the gas flow by a gas permeable separation means which forms a diffusion passage,
stimulating the gas situated in the measuring space with an electrically operated stimulating means at a reaction position, in order to effect a chemical reaction of a combustible gas-air mixture in the measuring space,
wherein the reaction position is disposed within the quenching distance, belonging to one combustible gas which is to be used, to the housing or the separation means,
wherein as stimulating means (8) is used:
- an electrical heating device with a resistive heating element through which electrical current flows, wherein the heating element is disposed within the measuring space at the reaction position,
or
- a plurality of constructively and galvanically separated electrodes which protrude at least partially into the measuring space, wherein the reaction position lies between the electrodes,
wherein the stimulating means is coupled with a voltage supply,
monitoring the measuring space with an optical detection device, the detection area of which is focused on the reaction position, in a spectral range around at least one first wavelength, wherein the intensity of the radiation is detected,
calculating the air ratio using the detected radiation intensities.

10. Method according to claim 9, wherein the air ratio is determined using a tabular classification from the measured intensity.

11. Method according to claim 9, wherein the electrical heating is adjusted such that the light radiation of the chemical reaction processes measured by the optical detecting device is maintained at a desired value.

12. Method according to one of claims 9 to 11, wherein the combustible gas-air ratio is iteratively altered and the energy supply to the stimulating means is diminished until a point is found which still allows a reaction operation with radiation emission, from which however in the direction of a lean mixture and a rich mixture in each case a collapse of the reaction takes place, in order to allot to this point an air ratio of the combustible gas-air mixture of λ=1.0.

## Revendications

1. Capteur servant à définir un coefficient d'air d'un mélange de gaz combustible-air (2),
avec un boîtier (3), qui délimite un espace de mesure (4),
dans lequel le boîtier (3) présente au moins sur un côté un passage de diffusion destiné à être couplé à un flux de mélange gaz combustible-air (2), dans lequel le passage de diffusion est formé par un agent de séparation (5) laissant passer le gaz,
dans lequel un moyen d'excitation (8) à fonctionnement électrique est compris dans l'espace de mesure (4) pour un apport en énergie pour provoquer une réaction chimique d'un mélange gaz combustible-air dans l'espace de mesure,
dans lequel
le moyen d'excitation (8) est réalisé et orienté pour entraîner l'apport en énergie au niveau d'une position de réaction se trouvant dans l'espace de mesure, qui est disposée à moins d'une distance d'extinction associée à un gaz combustible à utiliser par rapport au boîtier (3) ou à l'agent de séparation (5),
**caractérisé en ce que** le moyen d'excitation (8)
- est réalisé sous la forme d'un dispositif de chauffage électrique avec un élément de chauffage résistif traversé par le flux et dans lequel l'élément de chauffage est disposé à l'intérieur de l'espace de mesure au niveau de la position de réaction,
ou que le moyen d'excitation (8)
- est réalisé avec plusieurs électrodes séparées d'un point de vue structurel et galvanique, qui dépassent au moins en partie dans l'espace de mesure, dans lequel la position de réaction se situe entre les électrodes,
dans lequel le moyen d'excitation est couplé à une alimentation en tension,
dans lequel au moins un dispositif de détection (13, 14) optique est compris, dont la zone de détection dans l'espace de mesure (4) est orientée dans la position de réaction, dans lequel le dispositif de détection (13, 14) optique détecte l'intensité du rayonnement depuis la position de réaction dans au moins une première plage de longueurs d'onde et génère un signal (15) associé à l'intensité détectée, à partir duquel le coefficient d'air peut être déduit.

2. Capteur selon la revendication 1, dans lequel un dispositif de mesure (11, 12) est réalisé pour détecter de manière quantitative l'apport en énergie par le moyen d'excitation.

3. Capteur selon l'une quelconque des revendications précédentes, dans lequel le dispositif de détection optique présente un moyen sensible sur le plan optique.

4. Capteur selon la revendication 3, dans lequel le moyen sensible sur le plan optique est pourvu d'un filtre optique servant à sélectionner une plage de longueurs d'onde.

5. Capteur selon l'une quelconque des revendications précédentes, dans lequel un moyen de sélection optique est disposé entre la position de réaction et le dispositif de détection optique pour sélectionner le rayonnement sortant d'une zone partielle de la position de réaction aux fins de la détection par le dispositif optique.

6. Capteur selon la revendication 5, dans lequel le moyen de sélection optique est réalisé sous la forme d'un diaphragme ou sous la forme d'un guide de lumière ou sous la forme d'une lentille.

7. Capteur selon l'une quelconque des revendications précédentes, dans lequel une unité de régulation est couplée aux moyens d'excitation pour réguler l'apport en énergie dans l'espace de mesure, dans lequel la régulation est effectuée au moins en partie en fonction du signal du dispositif de détection optique.

8. Capteur selon l'une quelconque des revendications 2 à 7, dans lequel le dispositif de mesure est réalisé pour détecter l'apport en énergie avec au moins un capteur optique, qui surveille de manière optique le moyen d'excitation dans l'espace de mesure.

9. Procédé servant à définir le coefficient d'air d'un mélange gaz combustible-air, avec les étapes
de couplage d'un espace de mesure à un flux de gaz à surveiller, dans lequel l'espace de mesure est couplé au flux de gaz par un agent de séparation laissant passer le gaz, qui forme un passage de diffusion,
d'excitation du gaz se trouvant dans l'espace de mesure avec un moyen d'excitation à fonctionnement électrique au niveau d'une position de réaction pour provoquer une réaction chimique d'un mélange gaz combustible-air dans l'espace de mesure,
dans lequel la position de réaction est disposée à moins d'une distance d'extinction associée au gaz combustible à utiliser par rapport au boîtier ou à l'agent de séparation,
dans lequel on utilise en tant que moyens d'excitation (8) :
- un dispositif de chauffage électrique avec un élément de chauffage résistif traversé par un flux, dans lequel l'élément de chauffage est disposé à l'intérieur de l'espace de mesure au niveau de la position de réaction,
ou
- plusieurs électrodes séparées sur le plan structurel et galvanique, lesquelles dépassent au moins en partie dans l'espace de mesure, dans lequel la position de réaction se situe entre les électrodes,
dans lequel le moyen d'excitation est couplé à une alimentation en tension,
de surveillance de l'espace de mesure avec un dispositif de détection optique, dont la zone de détection est dirigée sur la position de réaction, dans une zone spectrale autour d'au moins une première longueur d'onde, dans lequel l'intensité du rayonnement sortant est déterminée,
de calcul du coefficient d'air à partir des intensités de rayonnement déterminées.

10. Procédé selon la revendication 9, dans lequel le coefficient d'air est déterminé à partir de l'intensité mesurée l'aide d'une association à un tableau.

11. Procédé selon la revendication 9, dans lequel le chauffage électrique est régulé de telle sorte que le rayonnement sortant de lumière mesuré par le dispositif de détection optique des processus de réaction chimiques est maintenu sur une valeur de consigne.

12. Procédé selon l'une quelconque des revendications 9 à 11, dans lequel le rapport gaz combustible-air est modifié de manière itérative et l'apport en énergie vers le moyen d'excitation est diminué jusqu'à trouver un point, qui permette encore un mode de réaction avec une émission de rayonnement, à partir duquel toutefois, respectivement un échec de la réaction ait lieu en direction d'un mélange plus maigre et plus gras pour attribuer audit point un coefficient d'air du mélange gaz combustible-air de λ = 1,0.
